# EUROPEAN PATENT APPLICATION

(11) **EP 4 491 129 A1**
(43) Date of publication of application: **15.01.2025**
(21) Application number: 23306213.2
(22) Date of filing: 13.07.2023
(51) Int. Cl.: A61B 8/12

(54) **APPARATUSES AND METHODS FOR CHARACTERIZING A DENTAL ULTRASONIC PROBE**

(71) Applicant: Carestream Dental LLC, Atlanta, GA 30339 (US)
(72) Inventor: CAPRI, Arnaud, 77435 MARNE LA VALLEE CEDEX 2 (FR)
(74) Representative: Casalonga

(57) **Abstract**

The present invention provides apparatuses and methods for characterizing an ultrasonic probe comprising a closed acoustic chamber, the acoustic chamber having an acoustic window and enclosing a transducer configured to emit an ultrasound signal and to receive an echoed ultrasound signal through the acoustic window. After having emitted an ultrasound signal in a plurality of directions and, for each direction, received a corresponding ultrasound signal reflected on a part of the acoustic chamber, characteristics of the acoustic chamber are determined from the received ultrasound signals.

## Description

### FIELD OF THE INVENTION

The present invention relates to the field of intraoral measurement methods and devices for the health care industry. Particularly, but not exclusively, the invention relates to characterizing an ultrasonic probe, for example, for improving its calibration and/or identifying degradations of its performance.

### BACKGROUND OF THE INVENTION

Ultrasound imaging has been adapted for intraoral use in a number of implementations and has been found to have a particular utility for tasks such as measurement of periodontal pocket depth. Conditions such as gingivitis, for example, can be detected by sensing the acoustic response of tissues.

Because of the non-emission of ionizing radiation, ultrasound imaging is inherently safer than ionizing methods and also allows repeating of an examination if needed. Ultrasound imaging can be used as a substitute for, or a complement to, various types of radiography (cone beam computed tomography or CBCT, panoramic x-ray, or intraoral x-ray imaging), magnetic resonance imaging (MRI), or nuclear medicine.

Ultrasound imaging may use high-frequency sound waves, typically between 1 to 100 MHz. High-frequency waves being more attenuated than low-frequency waves for a given distance but providing a higher resolution at short distances, high-frequency waves are suitable mainly for imaging superficial structures, e.g. for dermatology, or dental imaging. For example, high-frequency sound waves may preferably be between 10 to 50 MHz for periodontal pocket investigation. Conversely, low-frequency waves are suitable for imaging the deepest structures of the body. Moreover, image resolution is also improved with increasing wave frequency.

An ultrasound imaging apparatus generally comprises one or several transducers that act as ultrasound beam emitters and/or ultrasound beam receivers to receive echoes from the emitted signals. In addition, the ultrasound imaging apparatus may comprise various processing and display components used for generating and presenting images from acquired signals. An ultrasound beam emitter generates an ultrasound signal from an electrical signal and conversely, an ultrasound receiver generates electrical pulses from a mechanical ultrasound signal.

Objects in the path of emitted ultrasound signals return a portion of the ultrasound energy back to the transducer which generates electrical signals indicative of the detected structures. Transducers can be of different types among single element transducers or multi-element transducers (annular array, linear array, 2D array, etc.). Furthermore, when a multi-element transducer is used, the emission of ultrasound signals can be delayed in order to enable adaptive focusing. The electronic adaptive focusing makes it possible to increase the resolution depending on the depth of the imaged body structure. It also increases transducer sensitivity which improves image contrast.

To explore the different areas of the mouth of a patient while using a compact ultrasonic probe, the transducer may be movable in an acoustic chamber. Ultrasound signals are emitted according to different direction and/or position, using a reference position of the transducer. By sampling the echoed ultrasound signals, images representing the explored area of the mouth of the patient may be generated. The reference position of the transducer is generally determined by a sensor that may be associated with the transducer itself or with a motor controlling the movement of the transducer. Such a sensor presents drawbacks in terms of costs and compacity of the system.

In addition, the use of sensors requires a calibration that aims at associating acquisition windows with indexed positions of the transducer (or more generally of the motor controlling the movement of the transducer). However, calibration is time consuming and may need to be carried out multiple times during the product life. Indeed, depending on the usage condition (e.g., temperature and pressure), the system may undergo positioning deviation between the sensor and the real position of the transducer. Additionally, mechanical assembly tolerance and transducer manufacturing may lead to deviations between the active surface of the transducer and the housing of the transducer.

Therefore, there is a need for improvement, particularly to simplify calibration and to identify degradations of the probe.

### SUMMARY OF THE INVENTION

The present invention has been devised to address one or more of the foregoing concerns.

In this context, there is provided apparatuses and methods for characterizing a dental ultrasonic probe.

According to an aspect of the invention, there is provided a method for characterizing an ultrasonic probe comprising a closed acoustic chamber, the acoustic chamber having an acoustic window and enclosing a transducer configured to emit an ultrasound signal and to receive an echoed ultrasound signal through the acoustic window, the method comprising
emitting an ultrasound signal in a plurality of directions and, for each direction, receiving a corresponding ultrasound signal reflected on a part of the acoustic chamber; and determining characteristics of the acoustic chamber from the received ultrasound signals.

The method according to the invention makes it possible to obtain knowledge of characteristics of an ultrasonic probe that may be used for improving its calibration and/or identifying degradations of its performance.

In an embodiment, the ultrasound signal is reflected on an inner surface of the acoustic chamber, on an outer surface of the acoustic chamber, and/or on an internal member of the acoustic chamber, located between the inner and the outer surface of the acoustic chamber or on one of the inner and the outer surface of the acoustic chamber.

In an embodiment, the determined characteristics of the acoustic chamber comprise characteristics of the acoustic window.

In an embodiment, determination of characteristics of the acoustic window results from at least one of a thickness of the acoustic window that is different from a thickness of other portions of the acoustic chamber, an acoustic impedance of the acoustic window that is different from an acoustic impedance of other portions of the acoustic chamber, a given coating of the acoustic window, a frame of the acoustic window, and/or a specific curvature of a least a portion of the acoustic window.

In an embodiment, the method further comprises determining an acquisition window in the acoustic window

In an embodiment, the method further comprises determining at least one of a length, a width, and an orientation of the acquisition window with regard to a reference position of the transducer.

In an embodiment, the determined characteristics of the acoustic window comprise a distance or a signal time delay between an active surface of the transducer and the inner and/or an outer surface part of the acoustic chamber.

In an embodiment, the determined characteristics comprise an indication of a material heterogeneity between the inner surface part of the acoustic chamber and an active surface of the transducer.

In an embodiment, the method further comprises determining whether the ultrasonic probe may be used or not based on a characteristic of the material heterogeneity.

In an embodiment, the method further comprises estimating an intensity of the received echoed ultrasound signal, the echoed ultrasound signal being reflected on the inner surface of the acoustic chamber or on the internal member of the acoustic chamber, and comparing the estimated intensity with a stored reference intensity.

In an embodiment, the method further comprises comparing the difference between the intensities with a threshold.

In an embodiment, the transducer is movable in the acoustic chamber.

In an embodiment, the transducer is a 1D or 2D transducer array comprising selectable set of transducers, transducers being selected as a function of the determined characteristics of the acoustic windows.

In an embodiment, the method further comprises modifying a setting of the ultrasonic probe as a function of characteristics of the acoustic chamber.

In an embodiment, the method further comprises forewarning a user or practitioner of the ultrasonic probe about downgraded characteristics of the ultrasonic probe, as a function of characteristics of the acoustic chamber.

According to an aspect of the invention, there is provided an ultrasonic probe comprising a closed acoustic chamber, the acoustic chamber having an acoustic window and enclosing a transducer configured to emit an ultrasound signal and to receive an echoed ultrasound signal through a portion of the acoustic window, the ultrasonic probe further comprising a processing unit configured for carrying out each of the steps of the method described above.

The ultrasonic probe according to the invention makes it possible to obtain knowledge of its characteristics, that may be used for improving its calibration and/or identifying degradations of its performance.

The ultrasonic probe may be a compact intraoral ultrasonic probe, for example a compact intraoral ultrasonic probe used for measuring periodontal depth pocket.

At least parts of the methods according to the invention may be implemented in a processing unit. Accordingly, the present invention may take the form of an entirely hardware embodiment, an entirely software embodiment (including firmware, resident software, micro-code, etc.) or an embodiment combining software and hardware aspects that may all generally be referred to herein as a "circuit", "module" or "system". Furthermore, the present invention may take the form of a computer program product embodied in any tangible medium of expression having computer usable program code embodied in the medium.

Since the present invention can be implemented in software, the present invention can be embodied as computer readable code for provision to a programmable apparatus on any suitable carrier medium. A tangible carrier medium may comprise a storage medium such as a floppy disk, a CD-ROM, DVD, a hard disk drive, a magnetic tape device or a solid-state memory device and the like. A transient carrier medium may include a signal such as an electrical signal, an electronic signal, an optical signal, an acoustic signal, a magnetic signal or an electromagnetic signal, e.g. a microwave or RF signal.

### BRIEF DESCRIPTION OF THE DRAWINGS

Embodiments of the invention will now be described, by way of example only, and with reference to the following drawings in which:
**Figure 1** is a perspective view of an example of an ultrasonic intraoral wireless handheld device, according to some embodiments of the invention;
**Figure 2** illustrates schematically a cross-section view of an acoustic chamber containing an oscillating transducer, the cross-section being perpendicular to the axis of rotation of the transducer;
**Figure 3** illustrates an example of a movement of a transducer for estimating characteristics of an ultrasonic probe;
**Figure 4** illustrates an example of steps of an auto-diagnostic method, for estimating characteristics of an ultrasonic probe;
**Figure 5** illustrates an example of an image of a portion of the inner surface of a transducer housing, generated during an auto-diagnostic method, used for determining the position of an acquisition window;
**Figure 6** illustrates an example of an image of a portion of the inner surface of a transducer housing, generated during an auto-diagnostic method, used for determining abnormalities in an acoustic path;
**Figure** 7 illustrates an example of an image of a portion of the inner surface of the transducer housing, generated during an auto-diagnostic method, used for refining the setting of an ultrasonic probe;
**Figure 8** illustrates an example of an echoed ultrasound signal, measured during an auto-diagnostic method, used for monitoring acoustic properties of an acoustic chamber of an ultrasonic probe; and
**Figure 9** is a schematic block diagram of a processing device for implementation of one or more embodiments of the invention.

### DETAILED DESCRIPTION OF THE INVENTION

The following is a detailed description of particular embodiments of the invention, reference being made to the drawings in which the same reference numerals identify the same elements of structure in each of the Figures.

In the drawings and text that follow, like components are designated with like reference numerals, and similar descriptions concerning components and an arrangement or interaction of components already described are omitted. Where they are used, the terms "first", "second", and so on, do not necessarily denote any ordinal or priority relation, but may simply be used to more clearly distinguish one element from another, unless specified otherwise.

In the context of the present disclosure, the terms "operator" and "user" are considered to be equivalent and refer to the practitioner, technician, or other person who operates the ultrasonic probe.

In the context of the present disclosure, the phrase "in signal communication" indicates that two or more devices and/or components are capable of communicating with each other via signals that travel over some type of signal path. Signal communication may be wired or wireless. The signals may be communication, power, data, or energy signals. The signal paths may include physical, electrical, magnetic, electromagnetic, optical, wired, and/or wireless connections between the first device and/or component and second device and/or component. The signal paths may also include additional devices and/or components between the first device and/or component and second device and/or component.

In the context of the present disclosure, the term internal member refers to a phantom having specific acoustic properties, dimensions, sizes, geometries.

The term "subject" refers to the gums and other intraoral soft tissues (and possibly to tooth surfaces) of a patient that is being imaged.

According to some embodiments, images generated from data acquired by a transducer of an ultrasonic probe, during an auto-diagnostic method or when using the ultrasonic probe, are used to obtain characteristics of the ultrasonic probe. These characteristics may be used to calibrate the ultrasonic probe, to adjust settings, and/or to identify degradations of the performance of the ultrasonic probe. For example, such characteristics include the following ones:
- position or orientation of an acquisition window (e.g., angle, position, length, etc.),
- acoustic properties of members of the acoustic chamber comprising the transducer (e.g., position, size, and/or geometry of specific phantoms inserted in the housing or located on one of the inner and outer surface of the acoustic chamber, sensibility, impedance, and/or attenuation of the acoustic chamber, etc.),
- presence of abnormalities in the ultrasonic path (e.g., presence of air bubbles in the liquid or gel used to fulfill the acoustic chamber), and
- time delay (or distance) between the transducer and the housing.

The images used to obtain characteristics of the ultrasonic probe are obtained from data acquired while moving the transducer, for example, during an auto-diagnostic method or during acquisition of soft tissue information (e.g., by measuring the inner surface of the acoustic chamber, seamlessly to the user). The transducer may move linearly along a given path, may rotate around an axis, making complete turns (without angular oscillations, electrical contacts being provided, for example, by an electric slip ring) or partial turns (with angular oscillations), or moves according to any other given scheme. Alternately, a stationary array of transducers may be used.

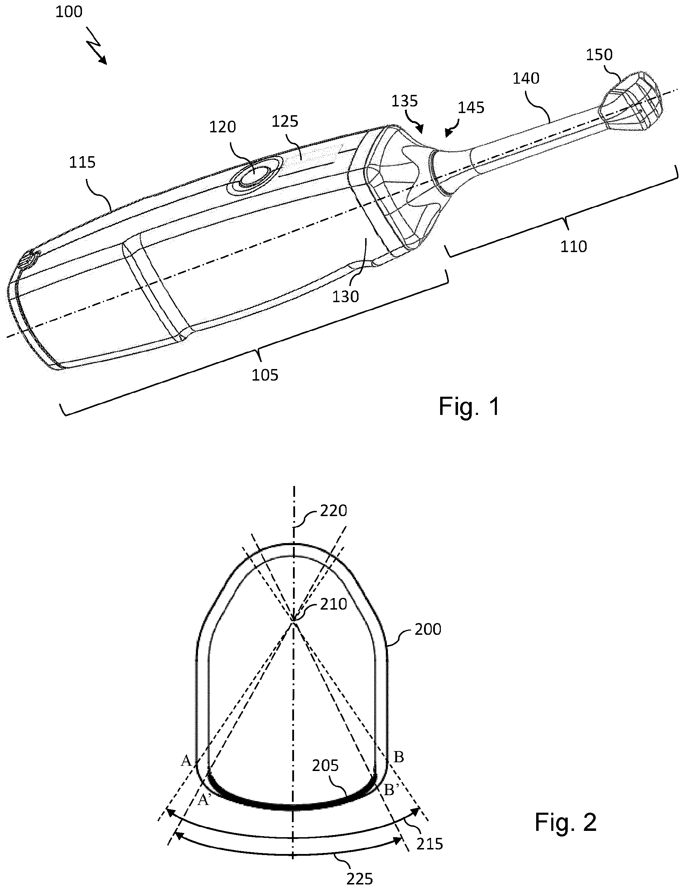

**Figure 1** is a perspective view of an example of an ultrasonic intraoral wireless handheld device 100, also denoted ultrasonic handpiece, handheld probe, ultrasonic probe, or probe, according to some embodiments of the invention. As illustrated, the ultrasonic intraoral wireless handheld device comprises a probe body 105 and a probe head 110 also called a transducer assembly.

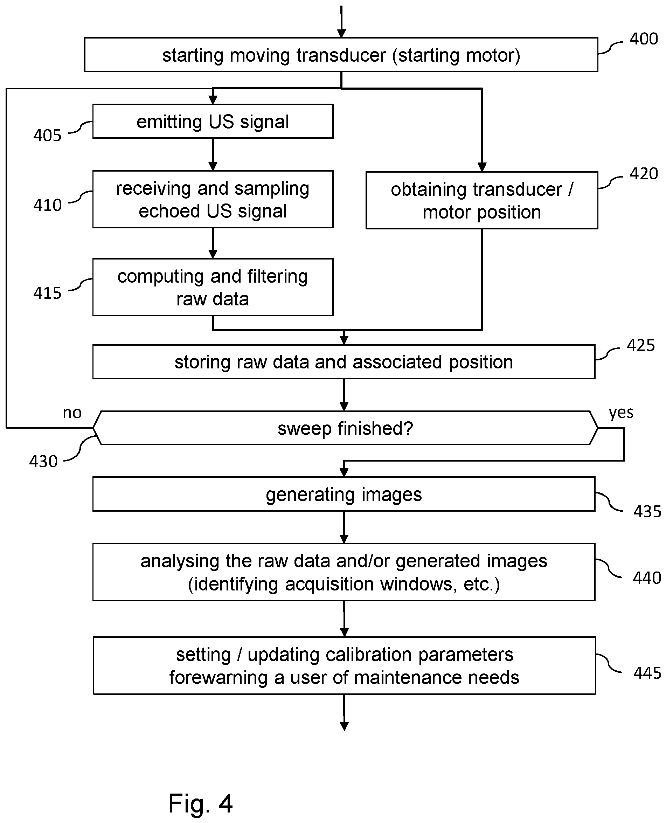

According to this example, probe body 105 comprises a housing 115 that may house a battery and electronic components (not represented), for example, a processing device configured to carry out steps as described by reference to Figures 4 to 8. In addition, probe body 105 comprises an interface making it possible for a user to get information from the probe and to provide instructions to the probe. For the sake of illustration, probe body 105 may comprise one or several buttons 120, a display 125, color LEDs (Light Emitting Devices) 130 (that are visible or located under the housing and visible through it when emitting light), a buzzer, an inertial measurement unit (not represented), and/or a haptic feedback vibrator (not represented). The inertial measurement unit makes it possible to detect user's actions like taps or double-taps, that may be used to interact with the ultrasonic probe. Using button 120 and short, long, and/or repeated clicks and/or using the inertial measurement unit, the user may browse through menus to get information from the probe and/or provide instructions to the probe. Probe body 105 also comprises mounting assembly 135 (located under a cover, which may be held in place by an elastic strap, and thus not visible) that makes it possible to mount a probe head to the probe body 105.

As illustrated, probe head 110 comprises a support arm 140 having mounting assembly 145 at one end (cooperating with mounting assembly 135 and thus, located under the same cover), so that probe head 110 may be mounted on a probe body, and a transducer chamber 150 at the other end (also denoted acoustic chamber or transducer housing). Transducer chamber 150 houses one or several transducers that may be stationary or that may move in the acoustic chamber, for example that may oscillate, to emit ultrasound waves toward an object and measure the echo. The transducer is wirelessly connected to electronic components of the probe body or is connected to these electronic components through wires, for example and not limitation, coaxial wires. According to some embodiments, the transducer is mounted at one end of a shaft (not represented) that extends longitudinally through the support arm. A cam may be mounted on the other end of the shaft so as to control the movement of the transducer using a motor located in the probe body. In addition, the shaft may be hollow to allow the passage of a coaxial wire connecting the transducer to electronic components of the probe body.

As described above, the acoustic chamber may house one or several transducers. These transducers may be of different nature. For example, the one or several transducers may be stationary or moveable, the transducers may be a linear 1D or 2D array of transducers or an annular array of transducers, or the transducers may use the same transducers for emitting ultrasound signals and for receiving ultrasound signals or may use different transducers for emitting ultrasound signals and for receiving ultrasound signals, etc. For the sake of clarity, the one or several transducers are generically referred to as a transducer in the following description.

According to the illustrated example, probe head 110 is a closed probe head having a transducer that is immersed in a sealed acoustic chamber filled with an internal acoustic coupling material such as a liquid or gel. In addition, the acoustic chamber may comprise solid coupling material that may be used, for example, to control the acoustic gradient of the acoustic chamber.

Ultrasonic probe 100 may be used for clinical applications such as periodontology, implantology, restorative works, and buccal dermatology.

In particular, ultrasonic probe 100 makes it possible to receive signals from which images of the soft tissues of a buccal space may be obtained, these images and/or signals enabling performing one or several of the following tasks:
- detecting morphological abnormalities such as gingival abscess and cancers, and/or
- taking buccal measurements such as measurements of the periodontal pocket depth, of the periodontal gingival margin, of the periodontal attachment level, of the width of an abscess, of anatomical marker distances, etc.
for example as described in WO2020148406 which is incorporated herein by reference in its entirety.

It is observed that these tasks may be carried out lively during the acquisition process (i.e., real time), or during a post exam operation, once the data set of images and/or raw signal are acquired.

According to some embodiments, imaging a portion of the inner surface of the transducer housing (or acoustic chamber), comprising one or more acoustic windows, and identifying the acoustic windows within the generated images, are used to determine a precise reference position of the transducer with regard to the acoustic windows and to define an acquisition window within the acoustic window, wherein reliable measurements may be done. It is observed that an acoustic window is a portion of the transducer housing designed to let ultrasound signals through (i.e., reflection of ultrasound signals on the acoustic window is reduced as much as possible). For the acoustic windows to be detectable, their thickness may be different from the thickness of the other portions of the housing, for instance, a fifth of the enclosure thickness. In addition or alternatively, the acoustic impedance of the acoustic windows may differ from the acoustic impedance of the other parts of the enclosure ( e.g., different materials). Still, in addition or alternatively, the acoustic windows may be detectable according to specific curvature of the acoustic windows or of their frame. A detectable frame surrounding the acoustic windows or a particular coating may also be used. Alternately or additionally, the material used to form the acoustic chamber may embed predetermined elements, acoustically detectable, that may be used as position references and/or as references to measure the intensity of echoed ultrasound signals. Such acoustically detectable elements behave like phantoms used to calibrate ultrasound probes.

By carrying out measurements of ultrasound signal echoed on the inner surface of the acoustic chamber, on the outer surface of the acoustic chamber (preferably by holding the probe away from any reflective surface), and/or acoustically detectable elements of the acoustic chamber, it is possible to identify acoustic windows, to determine the distance (or time delay) between an acoustic window and the active surface of the transducer, to determine the acoustic transmission quality of the material between an acoustic window and the active surface of the transducer, etc., which can be used to determine some characteristics of the ultrasonic probe. In turn, such characteristics may be used for calibrating the ultrasonic probe (e.g., defining acquisition windows, identifying the position of the transducer that corresponds to the borders of the acquisition windows, etc.), for identifying any degradation of its performances, for adjusting some parameters of the ultrasonic probe, dynamically or not, for example for adjusting the gain of the acoustic probe so as to compensate acoustic attenuation of a worn gel or for calibrating a set of ultrasonic probes so that they share the same characteristics, etc.

**Figure 2** illustrates schematically a cross-section view of an acoustic chamber containing an oscillating transducer, the cross-section being perpendicular to the axis of rotation of the transducer (which may be parallel to the longitudinal axes of the ultrasonic probe).

For the sake of clarity, only the housing 200 of the acoustic chamber is represented. According to the illustrated example, the acoustic chamber comprises one acoustic window 205 that acoustic characteristics are different from those of the other parts of the housing, for example its thickness is smaller than the one of the other part of the housing.

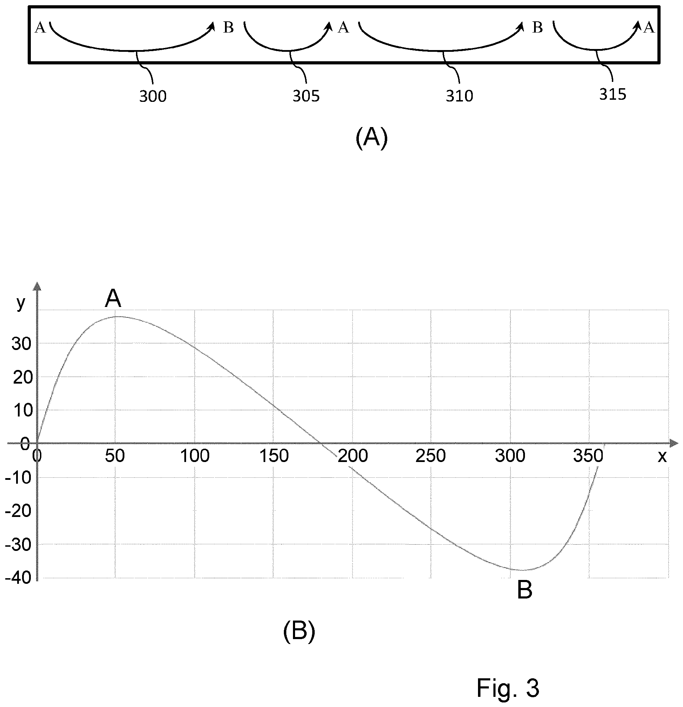

The transducer (not represented) may oscillate around axis 210 so as to emit ultrasound signals and receive corresponding echoed ultrasound signal within angular sector 215, delimited by angles A and B (for example from -38° to 38° with regard to axis 220 that is perpendicular to the acoustic window, in its center). Such an oscillating movement may result from a rotational mechanical crank comprising an eccentric or crank pin cooperating with a cam groove of a cam rigidly fastened to the transducer, as illustrated in Figure 3b.

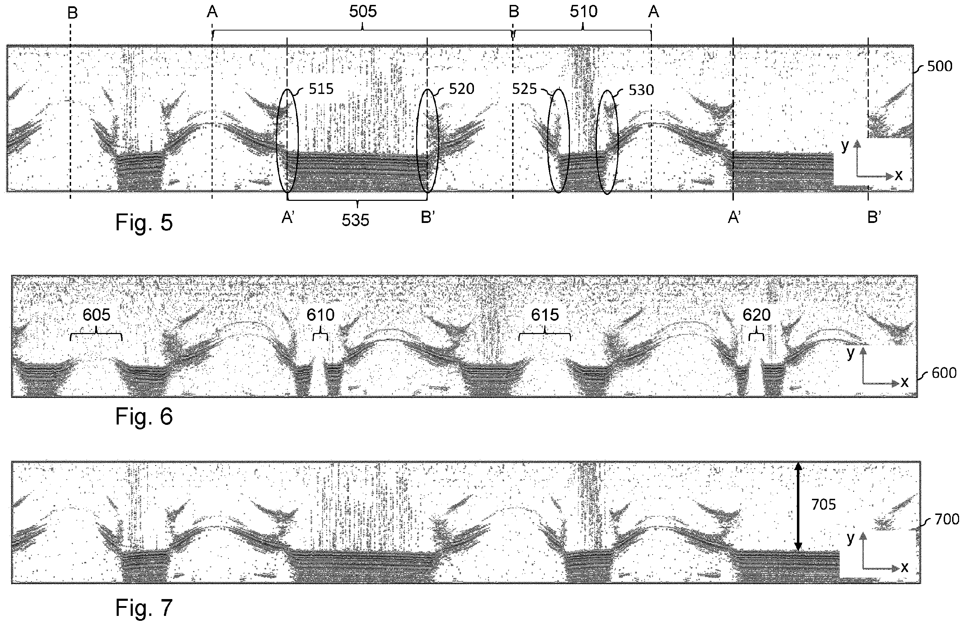

It is observed that to improve the accuracy of the measures done by the ultrasonic probe when examining a patient and to reduce complexity of processing the acquired data, sector 215 is swept twice, as illustrated in Figure 3a, and the echoed ultrasound signals are measured in one direction only. However, according to some embodiments of the invention, sector 215 is swept twice and the echoed ultrasound signals are measured in both directions, as illustrated in Figures 5, 6, and 7. Still according to some particular embodiments, sector 215 is swept twice and the echoed ultrasound signals are measured in both directions, the measures obtained in one direction being used for calibration or setting purposes and the measures obtained in the other direction being used for imaging soft tissue of a patient.

The measurements make it possible to estimate characteristics of the ultrasonic probe, these characteristics being used for calibrating the ultrasonic probe, updating settings, and/or for identifying degradations of its performances. According to some embodiments, calibration aims at identifying a reference position of the transducer and/or at estimating the position of acquisition windows. For example, an acquisition window 225 (represented with the curve in bold), delimited by angular positions A' and B', may be determined in sector 215 wherein measurement can be done, the acquisition window representing a trade-off between measurement coverage and measurement quality.

According to other embodiments, the transducer is moveable in translation or is stationary, the transducer being configured to emit and received ultrasound signals through the surface of the acoustic window, preferably the whole surface of the acoustic window.

**Figure 3** illustrates an example of a movement of a transducer for estimating characteristics of an ultrasonic probe.

As illustrated in Figure 3a, the transducer moves from angular position A to angular position B (phase 300) while carrying out high resolution measurements, next it moves faster from angular position B to angular position A (phase 305) while carrying out low resolution measurements or no measurement, next it moves again slower from angular position A to angular position B (phase 310) while carrying out high resolution measurements, and finally it moves faster from angular position B to angular position A (phase 315) while carrying out low resolution measurements or no measurement. It is noted that while no measurements are carried out during phases 305 and 315 when the ultrasonic probe is used to examine a patient, measurements are preferably carried out during these phases during an auto-diagnostic method. It is also noted that high resolution measurements may be done during phase 300 and 310 to examine a patient and low resolution measurements may be done during phase 305 and 315 for calibration or setting purposes. In such a case, the measurements done during phases 305 and/or 315 are not visible to the user. In addition, the measurements done during phase 305 and/or 315 may be used to improve the accuracy of the measurements done during phase 300 and/or 310 by adjusting dynamically some parameters of the probe.

Figure 3b illustrates the angular position of the transducer with regard to the angular position of the motor's output shaft. Rotation of the motor's output shaft makes the transducer oscillate. The angular position of the transducer is represented on the ordinate axis (y-axis) while the angular position of the motor is provided on the abscissa axis (x-axis).

An aspect of the auto-diagnostic method is to identify angular positions of the transducer with regard to the acquisition window (e.g., identifying the transducer angular positions corresponding to angular positions A' and/or B'), this position being expressed as a function of the corresponding position of the motor's output shaft that may be easily indexed (for example using a stepper motor, i.e., a motor that position may be commanded to move and hold one of several steps without any position sensor for feedback).

**Figure 4** illustrates an example of steps of an auto-diagnostic method, for estimating characteristics of an ultrasonic probe.

As illustrated, a first step of the method is directed to starting movement of the transducer (step 400), for example starting a motor making the transducer oscillate between two angular positions such as angular positions A and B in Figures 2 and 3.

Next, an ultrasound (US) signal is emitted (step 405) and the echoed ultrasound signal is received and sampled (step 410). Raw data representing the time for the emitted signal to be echoed and received are computed and may be filtered (step 415). For the sake of illustration, filtering the raw data may include ignoring raw data associated with ultrasound signals having time between emission and reception that is below a first threshold and/or above a second threshold, according to a temporal window of interest, that is to say raw data representing echoing surfaces that are too close and/or too far from the active surface of the transducer. According to some embodiments, only raw data associated with ultrasound signals having time between emission and reception above the second threshold (i.e., raw data representing echoing surfaces that are too far from the active surface of the transducer) are ignored. For example, the second threshold may be set in such a way as representing a distance of 1 cm between the active surface of the transducer and the echoing surface.

In parallel, the position of the transducer or of the motor's output shaft making the transducer oscillate, when emitting and receiving the ultrasound signal, is obtained (step 420).

This position and the raw data (possibly filtered) are stored (step 425).

Next, a test is carried out to determine whether the angular sector to be swept has been swept as requested (step 425), e.g., swept twice in a given direction. Steps 405 to 425 are repeated until all the measurements have been made.

When all the measurements have been made, an image is generated from the stored raw data (step 435), for example, using a known method. An example of a generated image is illustrated in Figure 5.

The generated image and/or the raw data are then analyzed (step 440) to determine some characteristics of the ultrasonic probe such as acoustic windows, abnormalities within the acoustic path between the transducer and its housing, etc.

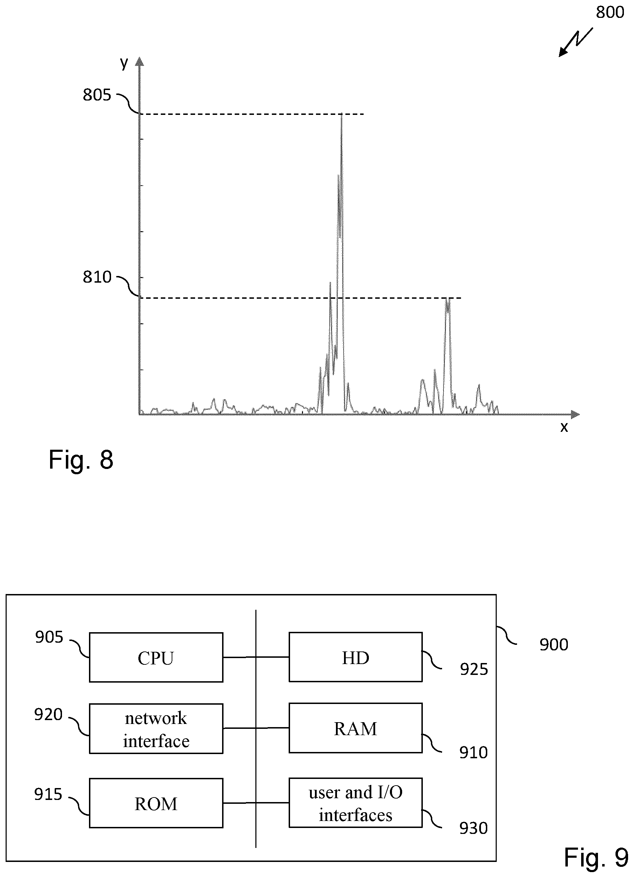

Analyzing a generated image for detecting and localizing an acquisition window is described by reference to Figure 5. Analyzing a generated image for detecting the presence of abnormalities such as air bubbles in an acoustic path is described by reference to Figure 6, and analyzing a generated image for refining the setting of an ultrasonic probe is described by reference to Figure 7. Analyzing ultrasound signals for monitoring acoustic properties is described by reference to Figure 8.

After having analyzed the generated image and/or the raw data, a calibration parameter of the ultrasonic probe may be set or updated (step 445). For example, the relative position of the acquisition windows with regard to the transducer or with regard to the motor's output shaft making the transducer oscillate may be set or updated. In addition, some indications may be provided to the user or practitioner, for example to forewarn the user or practitioner about the need for changing ultrasonic probe parts such as the probe head if it appears that the liquid or gel fulfilling the acoustic chamber is no longer efficient.

The steps illustrated in Figure 4 may be carried out upon request, for example upon a user's request, upon a request of a remote device (e.g., upon a request received from the provider/manufacturer (or other appropriate party) of the ultrasonic probe), or automatically, for example when starting or booting the ultrasonic probe.

According to some embodiments, the ultrasonic probe comprises a position sensor so that the steps illustrated in Figure 4 can be carried out only in particular positions of the probe, for example head up so (so that, for example, if air bubbles are present in the probe head, they can be detected), each time the ultrasonic probe is detected to be in a particular position, or on a periodic basis when detecting that the ultrasonic probe is in a particular position.

**Figure 5** illustrates an example of an image of a portion of the inner surface of a transducer housing, generated during an auto-diagnostic method, used for determining the position of an acquisition window.

According to the illustrated image, the ordinate axis (y-axis) represents the time delay between emission of an ultrasound signal and reception of the corresponding echoed ultrasound signal, which corresponds to the distance between the transducer and an echoing surface, that is to say the distance between the active surface of the transducer and the echoing surface. The abscissa axis (x-axis) represents the time at which the echoed ultrasound signal of which the time delay with the corresponding emitted ultrasound signal is represented on the abscissa axis, which represents a position of the transducer (or of the motor's output shaft that makes the transducer oscillate).

The image, referenced 500, is representative of the shape (represented four times because of the two sweeps of the transducer) of a portion of the inner surface of the transducer housing. The part 505 of image 500 represents the measurements that have been carried out between angular position A and angular position B while the part 510 represents the measurements that have been carried out between angular position B and angular position A. As apparent from the width of parts 505 and 510, the transducer is moving slower from angular portion A to angular position B than from angular portion B to angular position A (to improve angular resolution between angular portion A to angular position B).

Analyzing image 500 using, for example, a pattern analysis algorithm applied to the sum of pixel values along the y-axis, makes it possible to identify specific patterns representative of specific portions of the inner surface of the transducer housing.

For example, it is possible to take advantage of the cover/housing design to create strong acoustic signal deviations, in order to delimitate the acoustic window borders. Indeed, to be reflected, the acoustic ultrasound waves need to hit planar or slightly tilted surfaces. Therefore, as soon as the tilt of a surface becomes too high, the ultrasound signals are not echoed towards the transducer. Thus, it is possible to adapt the design of the acoustic windows to the transducer assembly so that the acoustic windows may be easily detected.

In case of a single element transducer or an annular multi-element transducer that is rotating or oscillating around a rotation axis, the acoustic window radius of curvature may be centered on the transducer rotation axis. In case of static 1-D or 2-D linear transducer array, the acoustic windows may follow the transducer array geometry (e.g., planar for a planar transducer array). In case of a 1-D or 2-D linear transducer array that is rotating or oscillating around a rotation axis, the acoustic window radius of curvature may be centered on the transducer rotation axis to obtain 3D acoustic volumes.

For example, the strong acoustic signal deviations referenced 515, 520, 525, and 530, make it possible to identify acoustic windows. Combined with the shape of the represented signals (e.g., flat portions) and the width of some particular shapes (e.g., width of the flat portions) between these strong acoustic signal deviations, the acquisition window 535, located between strong acoustic signal deviations 515 and 520, corresponding to a large and substantially flat portion, may be determined. By identifying the portion 535 in image 500, borders A' and B' of the acquisition window and their abscissa may be determined. Using the position of the transducer (or of the motor making the transducer oscillate) stored at step 420 in Figure 4 and the abscissa corresponding to borders A' and B', the position of the transducer (or of the motor) at which it should start measuring and its position at which it should end measuring may be determined. These start and end measuring angular positions may be stored as calibration data to be used as an acquisition window when examining a patient.

It is observed here that if the acoustic window borders may be delimited by a strong tilt or angulation in the head covers (to use the acoustic beam deviation or reflection properties), other solutions exist such adding landmarks (e.g., grooves), phantoms, or markers or by modifying the acoustic properties of the acquisition windows with regard to the other portions of the transducer housing, for example using specific textures, material, or coating.

When the transducer is a stationary array of transducers, the acquisition window makes it possible to determine which transducers of the array are to be active.

**Figure 6** illustrates an example of an image of a portion of the inner surface of the transducer housing, generated during an auto-diagnostic method, used for determining abnormalities in an acoustic path.

Image 600 is similar to image 500. It is representative of the shape of a portion of the inner surface of the transducer housing and corresponds to two sweeps (forward and backward). It is used to detect the presence of defects such as air bubbles, surface damages, wearing or degradation of the internal couplant, suspended particles, etc., in the acoustic chamber.

As apparent from the measures taken between angle positions A' and B' (and between angle positions B' and A'), an air bubble is located in the middle of the acquisition window, as illustrated with references 605, 610, 615, and 620.

Like the determination of an acquisition window determination, identifying such an air bubble may be based on pattern recognition and/or by comparing the actual pattern with the expected pattern. For the sake of illustration, an air bubble may be identified by identifying a hole in the image (i.e., because the air of the bubble does not reflect acoustic waves). The size of the air bubble may be determined by measuring the size of the hole. Such a size may be compared with one or more thresholds to determine whether the air bubble may be ignored, may be considered as acceptable, or should be considered as a significant issue.

**Figure 7** illustrates an example of an image of a portion of the inner surface of the transducer housing, generated during an auto-diagnostic method, used for refining the setting of an ultrasonic probe.

Image 700 is similar to or the same as image 500. It is representative of the shape of a portion of the inner surface of the transducer housing and corresponds to two sweeps (forward and backward). It is used to carry out some measurements, for example determining the distance or time delay between the active surface of the transducer and the inner surface of the transducer housing, as illustrated with references 705, and/or the outer surface of the transducer housing. Determining such distances, that may vary from one ultrasonic probe to another due to manufacturing reasons or inaccuracies, makes it possible to determine the time delay that should be set between exciting the transducer and sampling the echoed signal to avoid imaging the transducer housing and to harmonize transducer manufacturing (e.g., to set the same field of view in the soft tissues to all the probes of a set of probes).

**Figure 8** illustrates an example of an echoed ultrasound signal, measured during an auto-diagnostic method, used for monitoring acoustic properties of an acoustic chamber of an ultrasonic probe.

While imaging the inner surface of the transducer housing as described above, it is possible to monitor the evolution over time of the signal reflected by the inner surface.

According to some embodiments, the intensity of the ultrasound signal that is reflected by the inner surface of the transducer housing is measured and stored, at the time of manufacturing the ultrasonic probe (or at another time). It may be stored in a memory of the ultrasonic probe itself and/or in a memory or other data storage device of a nearby local computer/device or of a remote server. Next, each time the ultrasonic probe is turned on (or on request or according to other criteria, e.g., once a month), the intensity of the signal that is reflected by the inner surface of the transducer housing is measured and compared to the intensity of the echoed signal stored at the time of manufacturing the probe (or at the other time).

Figure 8 represents a graph of the intensity (y-axis) of an echoed ultrasound signal over the time (x-axis). For example, an echoed ultrasound signal may have a first peak of intensity 805 corresponding to a first surface of reflection (e.g., the inner surface of the acoustic chamber) and a second peak of intensity 810 corresponding to a second surface of reflection (e.g., the outer surface of the acoustic chamber).

According to some embodiments, the intensity of the signal that is reflected by the inner surface of the transducer housing at the time of testing the ultrasonic probe and the intensity of the signal that is reflected by the inner surface of the transducer housing at the time of manufacturing the probe (or at the other time) is compared to a threshold.

Degradation of the probe head is detected if the difference, in absolute value, between the intensity of the signal that is reflected by the inner surface of the transducer housing at the time of testing the ultrasonic probe and the intensity of the signal that is reflected by the inner surface of the transducer housing at the time of manufacturing the probe (or at the other time) is greater than the threshold. Such degradation may be signaled to the user of the ultrasonic probe or to someone in charge of the maintenance of the ultrasonic probe, for example for requesting the change of the probe head.

For the sake of illustration, such degradation may result from the wear of the internal couplant (e.g., the liquid or gel used to fill the acoustic chamber) contributing to the ultrasonic signal absorption and/or from a reduction of the transducer performance (a decrease of the intensity of the emitted ultrasonic signal induces a decrease of the intensity of the reflected signal sampled by the transducer).

**Figure 9** is a schematic block diagram of a processing device for implementation of one or more embodiments of the invention, in particular for characterising an ultrasonic probe, as described by reference to Figures 4 to 8.

Processing device 900 comprises a communication bus connected to:
- a central processing unit 905, such as a microprocessor, denoted CPU;
- a random access memory 910, denoted RAM, for storing the executable code of the method of embodiments of the invention as well as the registers adapted to record variables and parameters necessary for implementing a method for training and/or running an automatic tooth charting system according to embodiments of the invention, the memory capacity of which can be expanded by an optional RAM connected to an expansion port for example;
- a read-only memory 915, denoted ROM, for storing computer programs for implementing embodiments of the invention;
- a user interface and/or an input/output interface 930 which can be used for receiving inputs from a user, displaying information to a user, and/or receiving/sending data from/to external devices; and
- a network interface 920 typically connected to a communication network over which digital data can be transmitted or received for receiving/sending data from/to remote devices. The network interface 920 can be a single network interface, or composed of a set of different network interfaces (for instance wired and wireless interfaces, or different kinds of wired or wireless interfaces). Data packets are written to the network interface for transmission or are read from the network interface for reception under the control of the software application running in the CPU 905.

Optionally, the communication bus of computing device 900 may be connected to a hardware accelerator (e.g., an Artificial Intelligence accelerator module) and/or to a hard disk 925 denoted HD used as a mass storage device.

The executable code may be stored either in read-only memory 915, on hard disk 925 or on a removable digital medium such as for example a disk. According to a variant, the executable code of the programs can be received by means of a communication network, via the network interface 920, in order to be stored in one of the storage means of the computing device 200, such as hard disk 925, before being executed.

Central processing unit 905 is adapted to control and direct the execution of the instructions or portions of software code of the program or programs according to embodiments of the invention, the instructions being stored in one of the aforementioned storage means. After powering on, CPU 905 is capable of executing instructions from main RAM memory 910 relating to a software application after those instructions have been loaded from ROM 915 or from hard-disk 925 for example. Such a software application, when executed by CPU 905, causes the steps of the algorithms herein disclosed to be performed.

Any step of the algorithm herein disclosed may be implemented in software by execution of a set of instructions or program by a programmable computing machine, such as a PC ("Personal Computer"), a laptop computer, a tablet computer, a smartphone or similar device, a DSP ("Digital Signal Processor") or a microcontroller; or else implemented in hardware by a machine or a dedicated component, such as an FPGA ("Field-Programmable Gate Array") or an ASIC ("Application-Specific Integrated Circuit").

Although the present disclosure has been described herein above with reference to some specific embodiments, the present invention is not limited to these specific embodiments, and modifications will be apparent to a person skilled in the art which lie within the scope of the present invention.

Many further modifications and variations will suggest themselves to those versed in the art upon making reference to the foregoing illustrative embodiments, which are given by way of example only and which are not intended to limit the scope of the invention, that being determined solely by the appended claims. In particular, the different features from different embodiments may be interchanged, where appropriate.

In the claims, the word "comprising" does not exclude other elements or steps, and the indefinite article "a" or "an" does not exclude a plurality. The mere fact that different features are recited in mutually different dependent claims does not indicate that a combination of these features cannot be advantageously used.

## Claims

1. A method for characterizing an ultrasonic probe (100) comprising a closed acoustic chamber, the acoustic chamber having an acoustic window and enclosing a transducer configured to emit an ultrasound signal and to receive an echoed ultrasound signal through the acoustic window, the method comprising
emitting an ultrasound signal in a plurality of directions and, for each direction, receiving a corresponding ultrasound signal reflected on a part of the acoustic chamber; and determining characteristics of the acoustic chamber from the received ultrasound signals.

2. The method of claim 1, wherein the ultrasound signal is reflected on an inner surface of the acoustic chamber, on an outer surface of the acoustic chamber, and/or on an internal member of the acoustic chamber, located between the inner and the outer surface of the acoustic chamber or located on one of the inner and outer surface of the acoustic chamber.

3. The method of claim 1 or claim 2, wherein the determined characteristics of the acoustic chamber comprise characteristics of the acoustic window.

4. The method of claim 3, wherein determination of characteristics of the acoustic window results from at least one of a thickness of the acoustic window that is different from a thickness of other portions of the acoustic chamber, an acoustic impedance of the acoustic window that is different from an acoustic impedance of other portions of the acoustic chamber, a given coating of the acoustic window, a frame of the acoustic window, and/or a specific curvature of a least a portion of the acoustic window.

5. The method of claim 3 or claim 4, further comprising determining an acquisition window in the acoustic window.

6. The method of claim 5, further comprising determining at least one of a length, a width, and an orientation of the acquisition window with regard to a reference position of the transducer.

7. The method of claim 3 or of any one of claims 4 to 6 depending on claim 3, wherein the determined characteristics of the acoustic window comprise a distance or a signal time delay between an active surface of the transducer and the inner and/or an outer surface part of the acoustic chamber.

8. The method of any one of claims 1 to 7, wherein the determined characteristics comprise an indication of a material heterogeneity between the inner surface part of the acoustic chamber and an active surface of the transducer.

9. The method of claim 8, further comprising determining whether the ultrasonic probe may be used or not based on a characteristic of the material heterogeneity.

10. The method of claim 2 or of any one of claims 3 to 9 depending on claim 2, further comprising estimating an intensity of the received echoed ultrasound signal, the echoed ultrasound signal being reflected on the inner surface of the acoustic chamber or on the internal member of the acoustic chamber, and comparing the estimated intensity with a stored reference intensity.

11. The method of claim 10, further comprising comparing the difference between the intensities with a threshold.

12. The method of any one of claims 1 to 10, wherein the transducer is movable in the acoustic chamber.

13. The method of any one of claims 1 to 11, wherein the transducer is a 1D or 2D transducer array comprising selectable set of transducers, transducers being selected as a function of the determined characteristics of the acoustic windows.

14. The method of any one of claims 1 to 13, further comprising modifying a setting of the ultrasonic probe as a function of characteristics of the acoustic chamber.

15. The method of any one of claims 1 to 14, further comprising forewarning a user or practitioner of the ultrasonic probe about downgraded characteristics of the ultrasonic probe, as a function of characteristics of the acoustic chamber.

16. A computer program product for a programmable apparatus, the computer program product comprising a sequence of instructions for implementing each of the steps of the method according to any one of claims 1 to 15 when loaded into and executed by the programmable apparatus.

17. An ultrasonic probe comprising a closed acoustic chamber, the acoustic chamber having an acoustic window and enclosing a transducer configured to emit an ultrasound signal and to receive an echoed ultrasound signal through the acoustic window, the ultrasonic probe further comprising a processing unit configured for carrying out each of the steps of the method according to any one of claims 1 to 15.
